# EUROPEAN PATENT APPLICATION

(11) **EP 1 980 224 A2**
(43) Date of publication of application: **15.10.2008**
(21) Application number: 08007224.2
(22) Date of filing: 11.04.2008
(51) Int. Cl.: A61F 5/01, G01B 11/25

(54) **System and method for evalutating the needs of a person and manufacturing a custom orthotic device**

(30) Priority: 11.04.2007 US 911104 P
(71) Applicant: Hanger Orthopedic Group, Inc., Bethesda, MD 20814 (US)
(72) Inventor: Busch, Kaia, Forest Park, WA 98155 (US)
(74) Representative: Grünecker, Kinkeldey, Stockmair & Schwanhäusser Anwaltssozietät

(57) **Abstract**

A system for providing a custom orthotic can include a scanner, and imager for providing a digital three-dimensional image based on the scan, a gait and pressure measuring device and a data inputting system for inputting information regarding the customer. An analysis device can be provided to make modifications to the three-dimensional image based on the customer information, and the modified three-dimensional image can be forwarded electronically to a manufacturer for production.

## Description

### CROSS-REFERENCE TO RELATED APPLICATIONS

This application claims the benefit of U.S. Provisional Patent Application Serial Number 60/911,104 filed on April 11, 2007, the contents of which is incorporated in entirety by reference herein.

### FIELD OF THE INVENTION

The invention relates generally to a system and method for imaging a body part, such as a foot, modifying the image according to the needs of the person, if necessary, and a method of manufacturing an orthotic device. The invention also relates to a system and method for conducting gait and pressure analyses, and utilizing the analyses with the image to determine and manufacture a personalized and or custom orthotic device.

### BACKGROUND

Various systems and methods are available for providing custom orthotics to a person. However, the devices, systems and/or processes involved can be cumbersome and inefficient. Accordingly, there exists a need to provide a system and method for providing custom orthotics to a person more easily and efficiently.

### SUMMARY OF THE INVENTION

Generally speaking, the present invention is directed towards a system and method for producing a custom orthotic device for a person based on a three-dimensional image of a person's and/or client's anatomy such as but not limited to the foot and ankle complex together with gait and pressure information of the person. Preferably, the anatomical information is collected and processed at a remote station. The resulting data can be stored electronically, printed and provided to the person who can have an orthotic device manufactured accordingly. Alternatively, the person's data can be electronically transmitted to an orthotic device manufacturer for manufacture of the custom orthotic device. In one aspect of the invention, the manufacturer can prepare a three-dimensional model and positive mold based on the person's data and form an orthotic on the mold to create the custom orthotic device for the person. In another aspect of the invention, the manufacturer can prepare a three-dimensional model based upon the person's data and directly fabricate a definitive custom orthotic device In accordance with an embodiment of the invention, the anatomical data can be modified, for example to incorporate specific biomechanical, anatomical and functional needs of the person.

More specifically, the present invention is directed towards a remote system that includes a laser scanner together with a gait and pressure device to capture person's data for manipulation and/or creation of a custom orthotic device. The system uses a laser scanner to scan the person's foot to create a three-dimensional digital representation of the foot. The system also performs gait and pressure analysis of the foot which can be evaluated in conjunction with the 3D image. By providing a remote station at which the person's foot can be scanned and gait and pressure data gathered and measured, and by further providing electronic data with respect to these measurements that can be manipulated and transmitted to the manufacturer for production, an embodiment of the invention provides quantitative data, modification and design features, fabrication, comparative analysis, and convenience to the person as well as the clinician preparing the custom orthotic device.

Accordingly, it is an object of the invention to provide a system and method for evaluating the needs of a person to produce a custom orthotic device.

It is another object of the invention to provide a system and method having a remote station for conducting the evaluation that is electronically linked to a separate manufacturing location.

Yet another object of the invention is to provide a system and method for transmitting data regarding a person electronically for the production of a custom orthotic device.

Other objects, features, characteristics and advantages of the present invention will become more apparent upon consideration of the following detailed description with reference to the accompanying drawings, all of which form a part of this specification.

### BRIEF DESCRIPTION OF THE FIGURES

For a fuller understanding of the invention, reference is had to the following description, taken in connection with the accompanying drawings, in which:

FIG. 1 is a perspective view of a receiving station in accordance with an embodiment of the invention;

FIG. 2A is a perspective view of a foot scanner in accordance with an embodiment of the invention;

FIG. 2B is a top planar view of a foot scanner in accordance with an embodiment of the invention;

FIG. 3 is a sample foot scan in accordance with an embodiment of the invention;

FIG. 4 is a sample foot scan in accordance with an embodiment of the invention;

FIG. 5 is a sample screen shot of a receiving station in accordance with an embodiment of the invention;

FIG. 6 is a sample screen shot of a receiving station in accordance with an embodiment of the invention;

FIG. 7 is a sample screen shot of a receiving station in accordance with an embodiment of the invention;

FIG. 8 a flow diagram illustrating a system in accordance with an embodiment of the invention;

FIG. 9 is a flow diagram illustrating a system in accordance with an embodiment of the invention;

FIG. 10 is a flow diagram illustrating a system in accordance with an embodiment of the invention;

FIG. 11 is a flow diagram illustrating a system in accordance with an embodiment of the invention;

FIG. 12 is a flow diagram illustrating a system in accordance with an embodiment of the invention; and

FIG. 13 is a flow diagram illustrating a system in accordance with an embodiment of the invention.

### DETAILED DESCRIPTION OF THE PREFERRED EMBODIMENTS

The invention relates generally to a system and method for determining the needs of a person for producing a custom orthotic device. The present invention also relates to a system and method for remotely obtaining a 3D (3 dimensional) image of a person's anatomy such as the foot, but not limited thereto, as well as obtaining gait and pressure information regarding the person. The information and image can be utilized to determine appropriate custom orthotic configuration and recommendation for the person, for example a custom foot orthotic device.

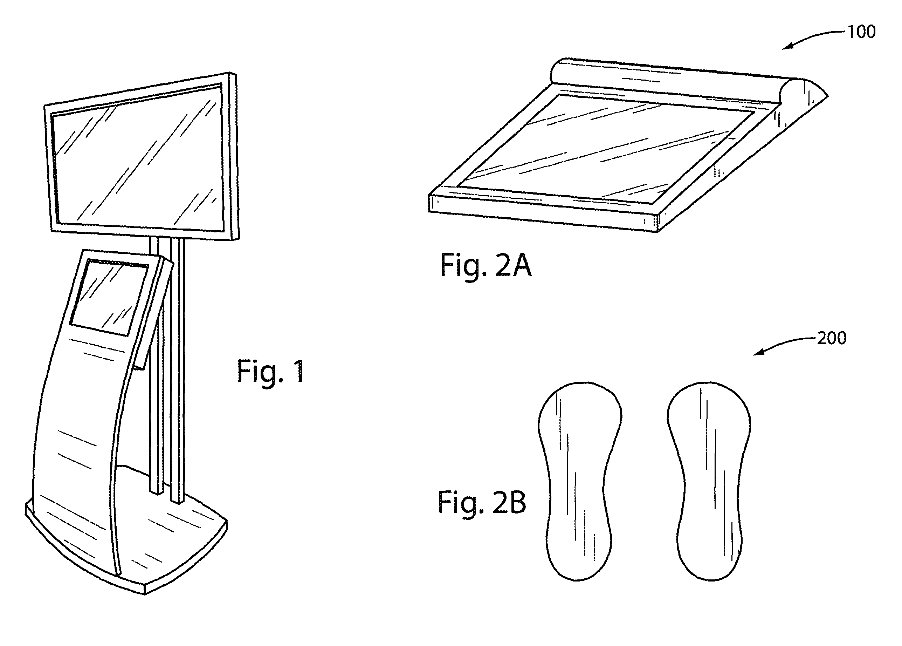

In accordance with an embodiment of the invention, a remote receiving station, such as a kiosk or a booth, is provided. FIG. 1 illustrates an exemplary kiosk in accordance with an embodiment of the invention. A person wishing to have a custom orthotic device prepared is guided to the remote station where the person's information is collected. For example, the person's shoe size, activity and clinical information can be entered into the system and saved as a data file for the particular person. The gait, pressure, and balance scans of the person's foot can also be collected at the station, preferably followed by an analysis of the three parameters (gait, pressure, and balance). This analysis can be augmented by utilizing 2D, 3D and real time video review.

Also at the station, a 3D scan of the person's foot can be taken, for example, using a laser scanner. The 3D scan is preferably evaluated in conjunction with the gait, pressure, and balance analysis in order to create a custom orthotic device. Accordingly, a custom orthotic configuration and recommendation for the person can be determined based on the data and the clinical/physical evaluation. At the kiosk, other features such as modifications to the 3D image, choice of material for the orthotic device, adaptations and special instructions can be added to the gathered information and analyses.

The station may also include a sensing platform on which the person can stand or walk or a sensing insert placed inside the person's shoe. Referring to FIG. 2A, an exemplary sensing platform 100 is shown. The platform can include a mat, which collects gait information of the person, and preferably pressure information as well. For example, the mat can include sensors, such as thin film tactile pressure and force sensors, which can monitor pressure and force of the foot placed thereon. An embodiment of the insert 200 for placement in the person's shoe is shown in FIG. 2B and utilizes a similar sensor configuration, such as the thin film tactile pressure and force sensors to derive quantitative data relative to gait, pressure, and balance analysis during activities such as but not limited to walking, running, sporting activities, and normal locomotion.

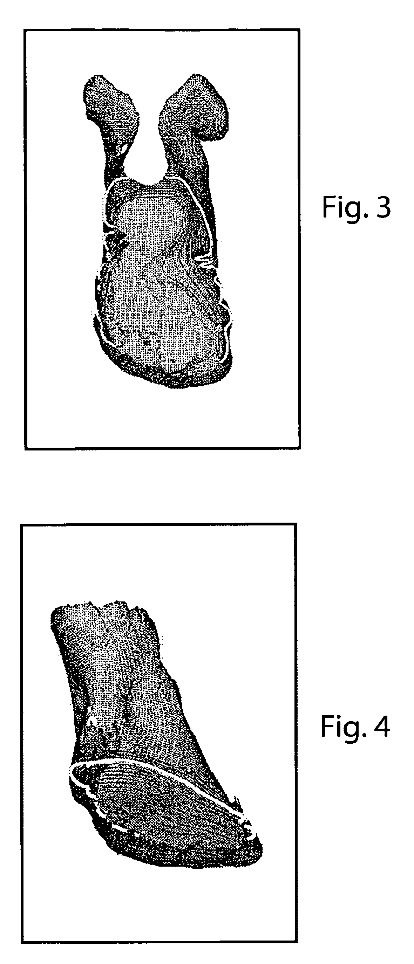

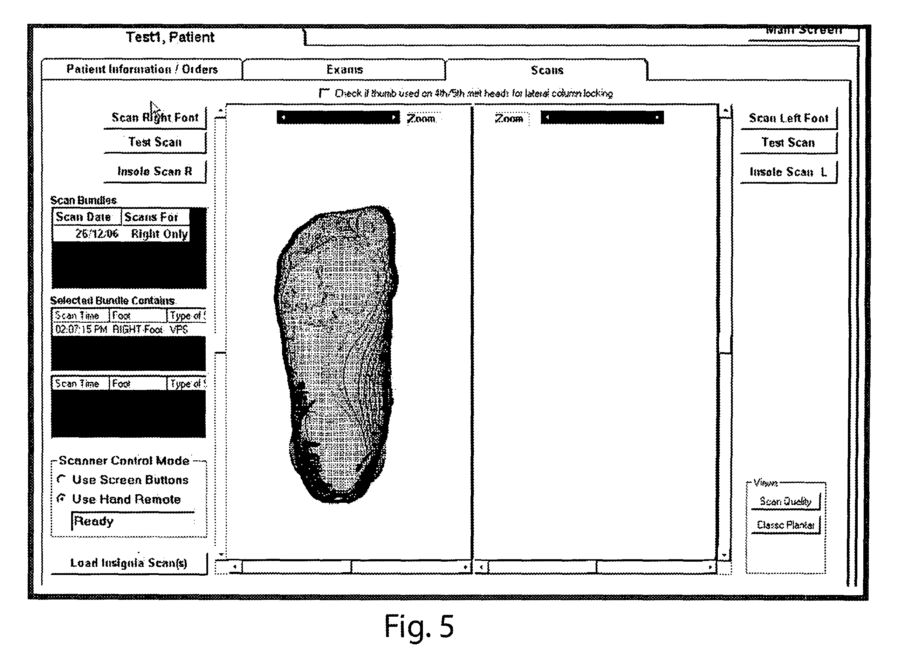

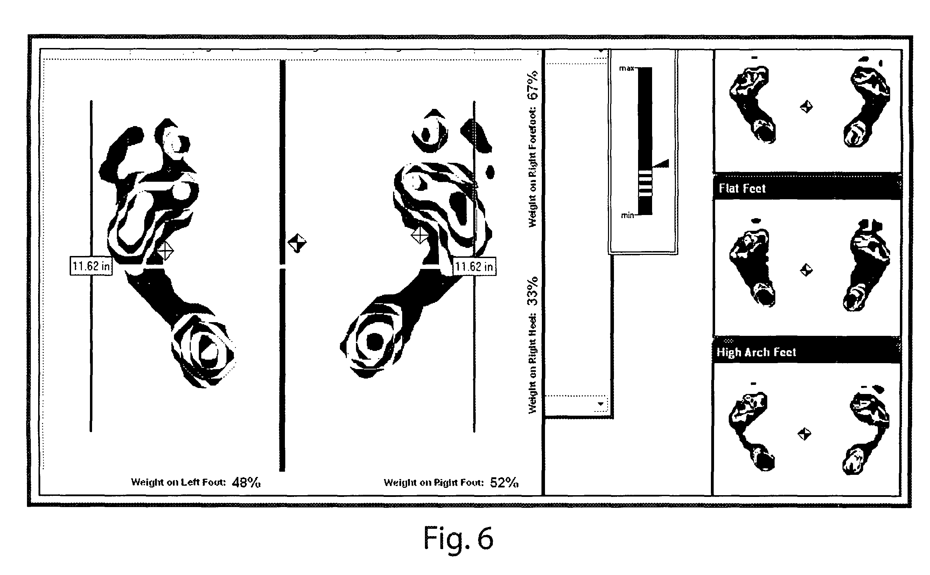

Examples of analysis and data provided by an embodiment of the invention includes, without limitation, a comparison of anatomical data and person presentation with respect to standard profiles of normal, flat and high arch feet to determine where they fall relative to the scale of normal, flat or high arch feet. The system preferably also records foot type, regions with highest pressure and the percentage of weight distribution either while standing or walking. For example, the system preferably determines the difference in weight distribution between the left and right feet, the forefoot versus the rearfoot, and the right side versus the left side. FIGS. 3-4 illustrate examples of foot scans that can be obtained at the receiving station, and FIGS. 5-7 illustrate examples of the information obtained and displayed at the receiving station in accordance with exemplary embodiments of the invention.

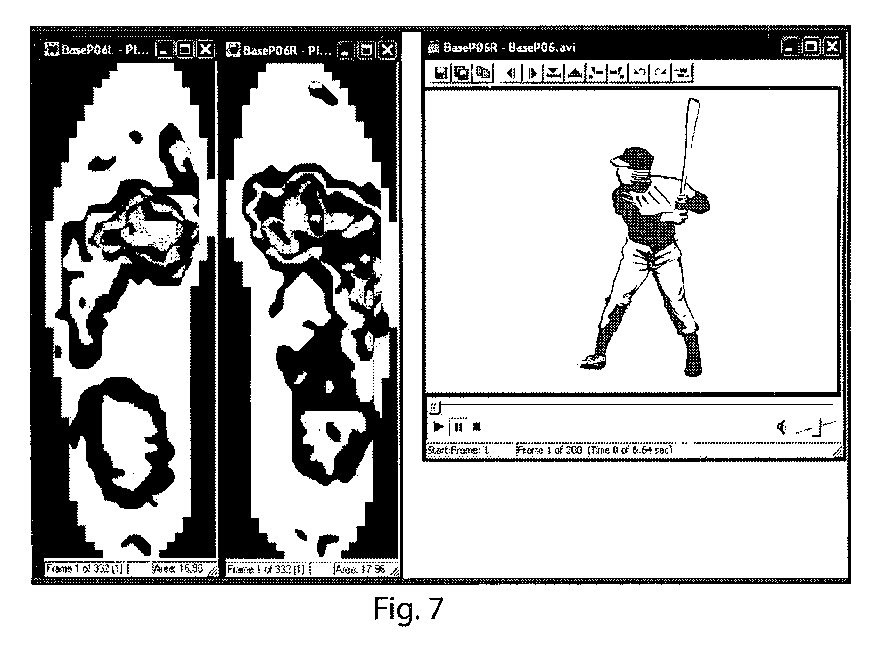

In accordance with an embodiment of the invention, the mat and in-shoe design configuration includes calibration capabilities which can facilitate generating true pressure measurements. The mat also has a smooth and a rigid surface, which can maintain the calibration of the system, accuracy of the output and safety of the person during the gait cycle. The mat can include a flat back or an alternative design without deviating from the scope of the invention. The mat can be relatively long to provide a longer gait pattern analysis.
Alternatively, a longer mat may be suitable to obtain data relative to sequential gait cycles and/or activities requiring a wider base of support. In addition, the in-shoe design may be utilized to obtain client data for pre-treatment, and post-treatment comparisons during normal locomotion and/or specified sporting activities, for example, running, climbing, skiing, and cycling. Referring to FIG. 7, the client data during playing baseball, more specifically, while the client is at bat, can be obtained.

In accordance with an embodiment of the invention, the platform mat and in-shoe design includes video synchronization capabilities, thus being able to accept video data, by way of non-limiting example, in .avi or .mpeg format, to synchronize it to the gait and pressure data. A sway analysis assembly is provided for analyzing the person's sway and documenting the values for their equilibrium, before and after treatment. Thus, the platform can measure the progression of force of the feet while walking.

This collective data including the analyses can be stored electronically, in hard copy format, and can be printed and provided to the person for their reference. The clinician can review the printed data and either fabricate the orthotic device through an internal fabrication site or send it to an orthotic manufacturer to have an orthotic device produced in accordance with the data. Alternatively, the data can be transmitted to a manufacturer electronically, for example, via email. The manufacturer preferably reviews the data and processes the scans using computer aided design (CAD) or computer aided manufacturing (CAM) technology. CAD/CAM technology can be preferable because it permits digital storage of pre and post application of orthotic intervention, raw data manipulation of the files to incorporate the clinical and relevant data for fabricating the orthotic device, digital records for comparison views and tracking the person, and the ability to fabricate additional orthotic devices from the same digital file. The CAD/CAM systems that can be utilized in accordance with an embodiment of the invention include 2D grayscale conversion imaging often referred to as "shape from shading" and 3D laser imaging.

The remote station, or kiosk, also includes a laser scanner to obtain a 3D image of the person's anatomy, for example the foot. By way of non-limiting example, a laser scanner can obtain a 3D image as follows. The laser scanner emits a laser light, which is passed over the person's foot. The person's foot reflects the laser light back to a receiver. The receiver then measures the time or phase difference between the transmission of the laser light and its reception by the receiver. This time or phase difference is used to calculate the surface anatomy of the person's foot. Additionally, a pulse of light can be time stamped within the software to provide differentiation between points of reference.

A 3D image of a person's foot can also be obtained using a 2D (two-dimensional) grayscale conversion imaging, wherein the shade variations of a 2D shape and model are used to create a 3D image or object. More specifically, a computer program can convert the 2D shape and a fixed parameter to determine the relative height and width, and analyze the shadow effect on the surface of the object to determine the depth, thus creating a 3D image. The 3D scan and 2D grayscale conversion imaging are merely examples of methods for obtaining a 3D image. It is to be understood that other ways of obtaining a 3D image can be utilized without deviating from the scope of the invention.

In accordance with a preferred embodiment of the invention, a 3D image is derived, either from the 2D or 3D system, and using CAD tools, the image is modified if necessary, for example, to address certain anatomical and biomechanical needs of the person, for example, based on clinical and physical evaluation, the gait, pressure, and balance analysis. Once the modifications, if any, are complete, a model can be produced from the 3D image and other data and exported to the carver software. The carver software can fabricate a definitive model, and a model can be carved from a variety of material, such as foam, wood, or composite blanks. An orthotic can be fabricated over the carved model, for example, through material vacuum forming techniques and hand finishing. Alternatively, the definitive orthotic device can also be carved, similar to or in lieu of a model, as a matter of application specific design choice.

Preferably, data regarding the person is maintained at each step, thereby permitting a comparison between the original model, the modified model, and subsequent follow up scans, if any. This information can be utilized to document medical necessity, validate the effectiveness of a treatment, monitor the progress of the person and facilitate follow up of the person. The 3D scanning can substantially reduce the need for later impressions, which can be time consuming. 3D scanning may also reduce the need for foam box impressions that are often less, accurate and negative models that can be time and labor intensive.

Once the orthotic device is produced, it is sent to either the person or the station from which the request for the orthotic device was received. By way of non-limiting example, the station can be located within a retail store, a clinic, a physician's office or a hospital. The person is fit with the orthotic device and the clinician evaluates for proper fit, anatomical and biomechanical effectivity. The clinician can test the orthotic device and ensure that it is a proper fit. If there is a problem with the fit of the orthotic device, it can be modified on site or the raw scan and analysis data can be modified accordingly, the modified data can be transmitted to the manufacturer and a new orthotic device can be manufactured.

Certain embodiments of the invention can include a touch screen interface for both receiving certain information and displaying collected data. Examples of data entry interfaces include drop-down menus from which the person or a user conducting the scans can enter information. Certain information, such as shoe size, shoe insole previously or currently recommended and purchase history can be entered. Referring to FIGS. 5-8, a split screen can be provided to view the gait analysis and scanning information simultaneously. Therefore, the data from both can be interpreted simultaneously, which can enhance efficiency.

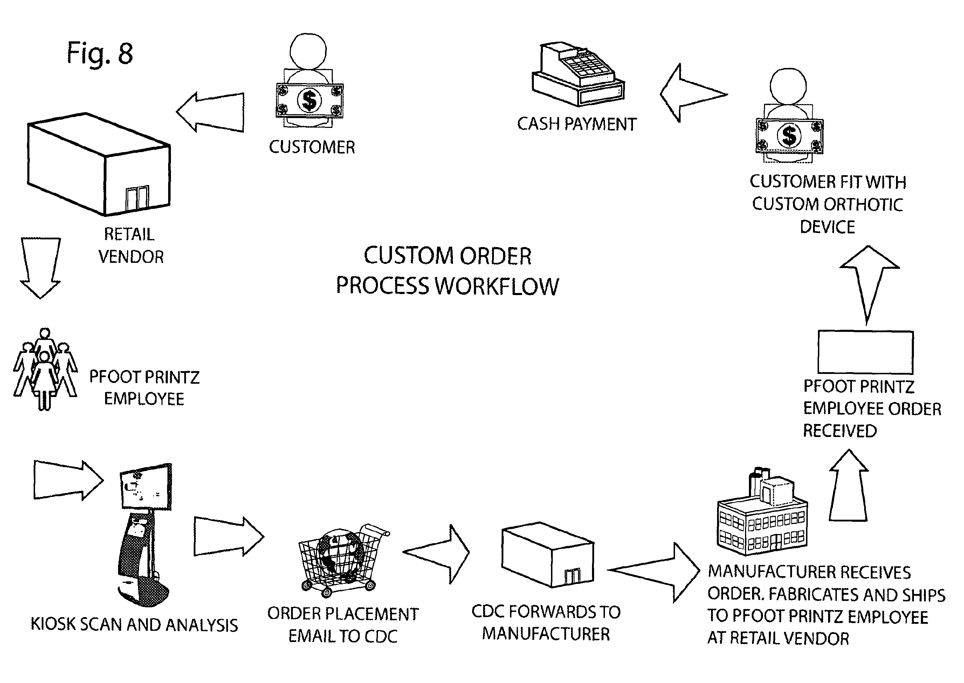

An exemplary process in accordance with an embodiment of the system is illustrated in FIG. 8, in which a flow diagram is provided describing the steps. As shown, a customer can go to a retail vendor and have an employee conduct the scans, for example, of the customer's feet. The feet can be scanned using a laser scanner to create a three-dimensional image or other representation of the customer's feet. A gait and pressure device can be used to obtain and analyze the customer's gait and pressure information in connection with the three-dimensional image. The employee can also enter information regarding the customer at a receiving station, such as a kiosk. The employee can then place an order for an orthotic by sending the gathered information and analysis via email to a designer at a central design center (CDC), where the designer can create a three-dimensional model and positive mold based on the scan and information. The designer can also modify the model or mold based on specific needs of the customer, for example, to incorporate biomechanical, anatomical and functional needs of the customer. The CDC can then forward the order and data gathered, including the modified model or mold to a manufacturer. The manufacturer can fabricate an orthotic in accordance with the order received and forward the orthotic to the employee at the retail vendor, who can fit the customer with the custom orthotic fabricated based on the customer's scan and information.

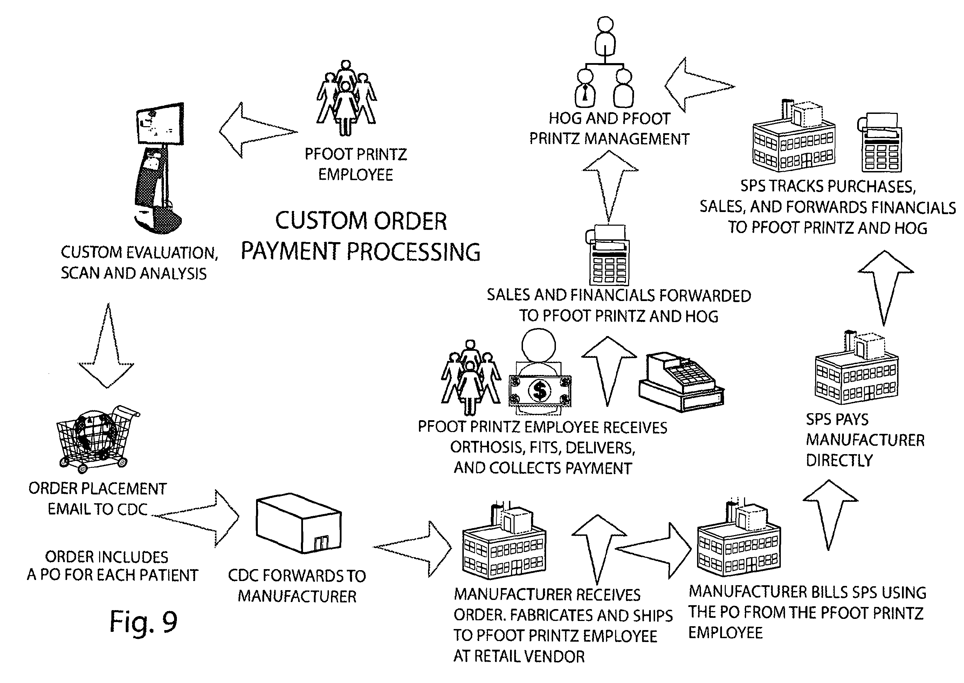

An example of the process of billing the customer for the custom orthotic is illustrated in FIG. 9. As shown, once the employee at the retail vendor places the order for the customer, the order can also include a purchase order for the customer. When the manufacturer fabricates and ships the custom orthotic to the retail vendor, the manufacturer preferably also forwards a bill to an entity, which pays the manufacturer. The entity can be, by way of non-limiting example, a subsidiary of a management company that oversees the system. Meanwhile, the employee at the retail vendor receives payment from the customer when delivering the orthotic to the customer. The sales and financial data can be forwarded from the retail vendor to the management, who also receives the purchase, sales, and financial data from SPS regarding the purchase order, payment, etc. The management can thus reconcile the data from the retail vendor and subsidiary.

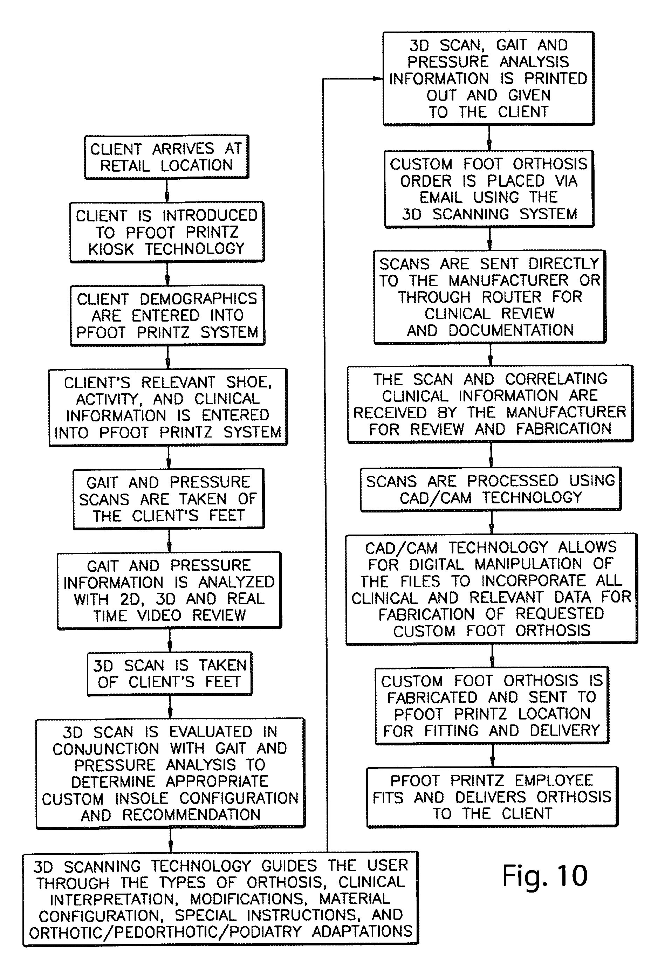

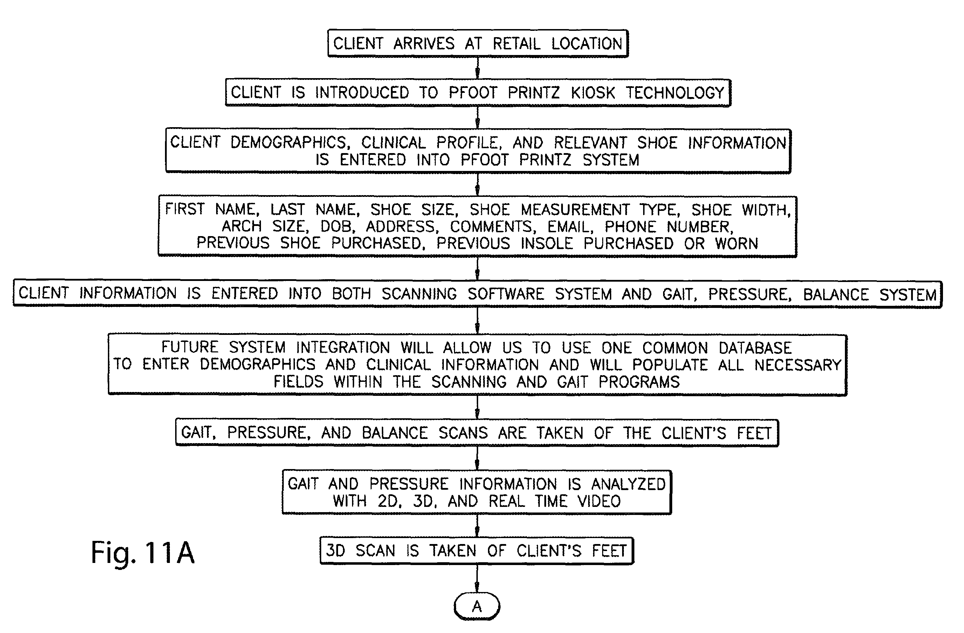

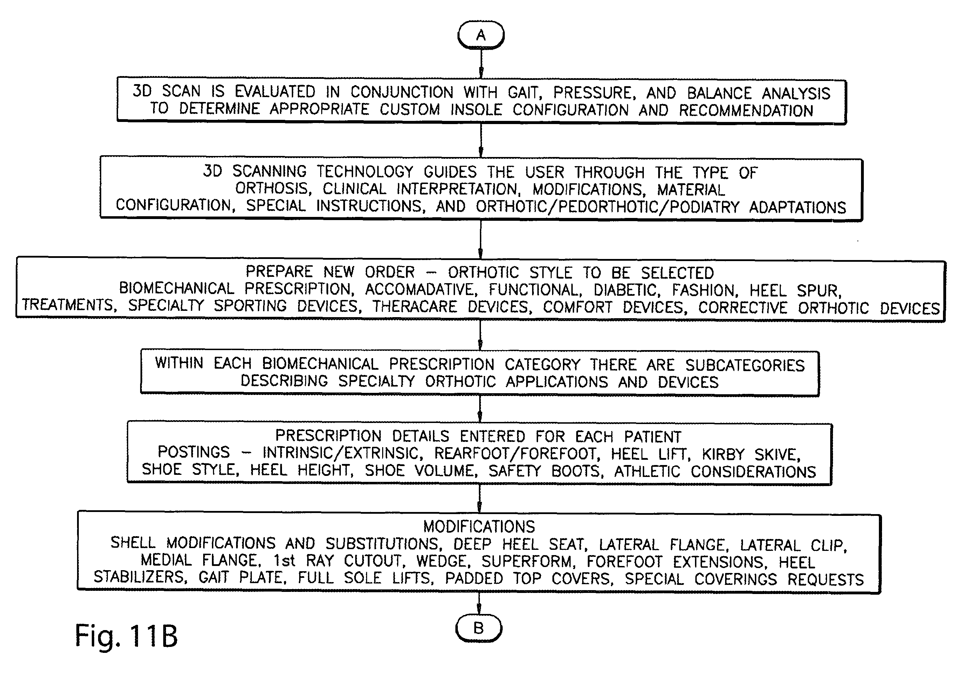

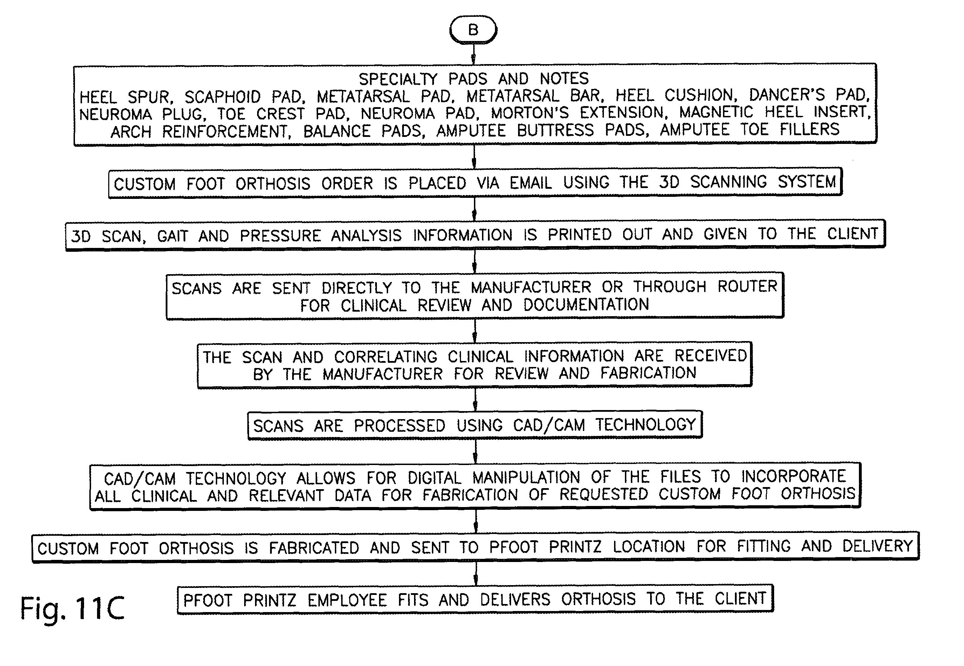

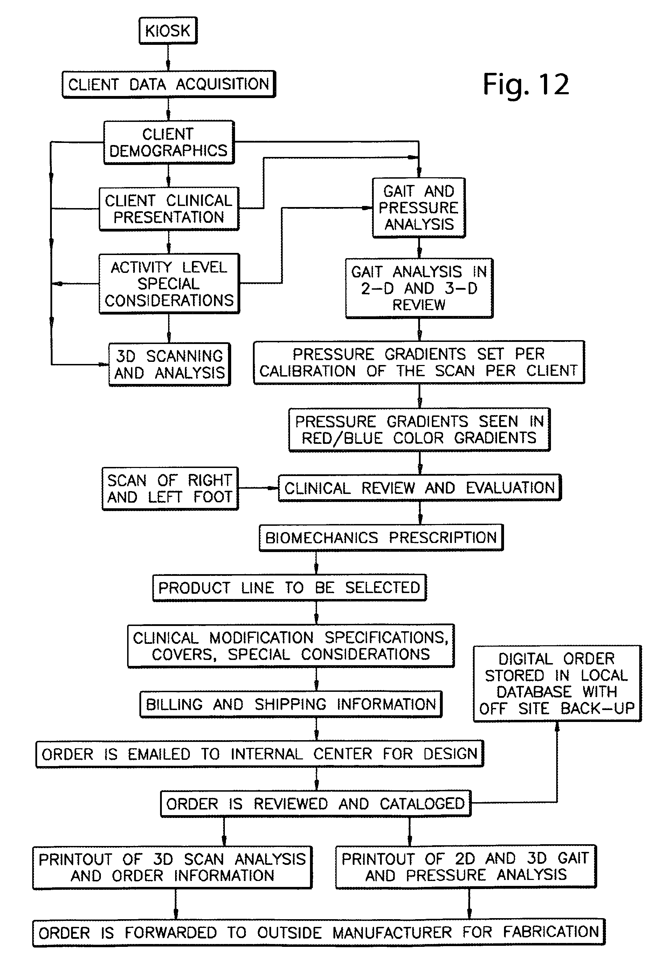

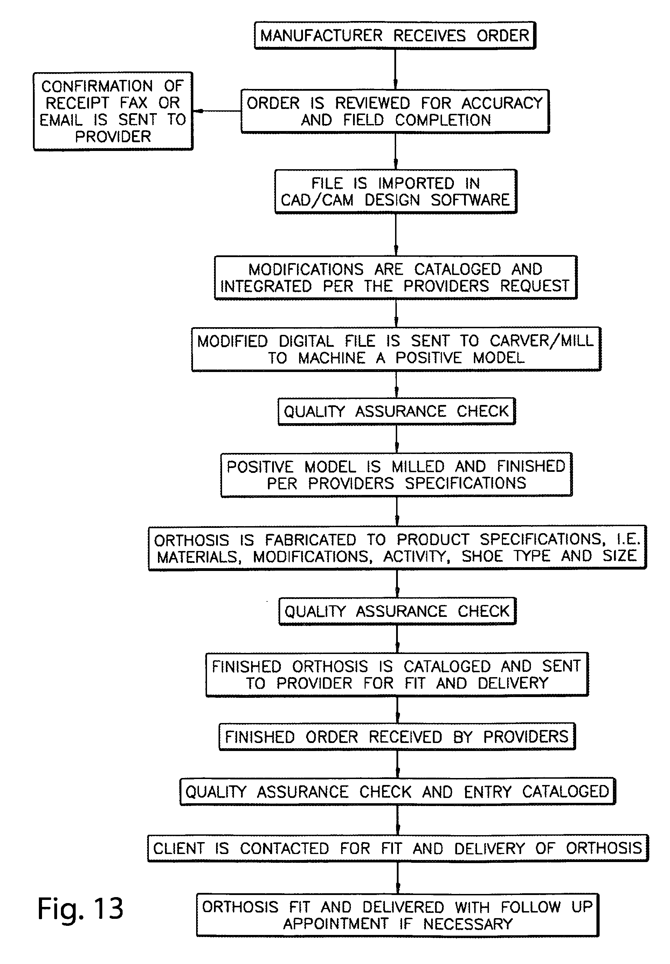

FIGS. 10-13 provides flow charts of additional examples of processes in accordance with certain embodiments of the invention. One of ordinary skill in the art would find FIGS. 10-13 to be self explanatory, and thus they will not be described in detail herein.

In accordance with an embodiment of the invention, the gait and pressure analyses can be provided for a person stepping on the platform with his/her shoes on or off. Alternatively, it may be preferable to permit the deletion of certain scans, such as 3D imaging and/or gait and pressure analysis. It may also be preferable to select which information from the collective data is sent to the manufacturer.

It will be understood that while fundamental novel features of the invention as applied to preferred embodiments and examples thereof have been described and pointed out, various omissions and substitutions and changes in the form and details of the disclosed invention may be made by those skilled in the art without departing from the spirit of the invention. It is the intention, therefore, to be limited only as indicated by the scope of the claims appended hereto.

## Claims

1. A system for providing a custom orthotic, the system comprising:
a laser scanner constructed and arranged to conduct a laser scan of a body part of a person;
an imager for receiving the laser scan of the body part of the person, the imager generating a digital three-dimensional image of the scanned body part;
a gait and pressure capturing device constructed and arranged to electronically obtain the gait and pressure information of the body part;
a data input device for receiving data regarding the person, including customization requests;
an analysis device for electronically receiving the three-dimensional image and the gait and pressure information of the body part;
the analysis device analyzing three parameters of the body part, the three parameters comprising gait, pressure and balance, and evaluating the three-dimensional image based on the three parameters; and
a customization device for digitally modifying the three-dimensional image based on the data regarding the person and the result of the evaluation.

2. The system of claim 1, wherein the gait and pressure capturing device comprises a plurality of sensors.

3. The system of claim 1, wherein the gait and pressure capturing device comprises a sensing platform on which the body part can be placed.

4. The system of claim 1, wherein the gait and pressure capturing device comprises a plurality of thin film tactile pressures and force sensors therein.

5. The system of claim 5, wherein the sensors are constructed and arranged to monitor pressure and force of the body part placed thereon.

6. The system of claim 5, wherein the sensors are constructed and arranged to derive quantitative data relative to gait, pressure, and balance of the person.

7. The system of claim 1, wherein the gait and pressure capturing device comprises a sensing insert constructed and arranged to be inserted into the person's shoe.

8. The system of claim 1, further comprising a model generator for producing a model of the body part.

9. The system of claim 1, further comprising a model generator for producing a model of the body part and an orthotic producer for producing an orthotic over the model.

10. The system of claim 1, further comprising:
a video device for capturing video data regarding the person; and
a synchronizing device for synchronizing the video data to the gait and pressure data.

11. The system of claim 1, further comprising a printing device for providing a hard copy of the gathered data.

12. The system of claim 1, further comprising a remote station wherein the laser scanner, the gait and pressure capturing device and the data input device are provided within a unitary station.

13. A system for providing a custom orthotic, the system comprising:
a scanner constructed and arranged to conduct a scan of a body part of a person;
an imager for receiving the scan of the body part of the person, the imager generating an electronic three-dimensional image of the scanned body part;
an information capturing device constructed and arranged to electronically obtain information on the person;
an analysis device for evaluating the three-dimensional image based on the information;
a model generator for producing a model of the body part based on the three-dimensional image; and
an orthotic producer for producing an orthotic over the model.

14. An three-dimensional imaging system for a custom orthotic, the system comprising:
a laser scanner constructed and arranged to conduct a laser scan of a body part of a person;
an imager connected to the laser scanner, the imager receiving the laser scan from the laser scanner and generating a digital three-dimensional image;
a screen for receiving the three-dimensional image from the imager, the screen further constructed and arranged to display the three-dimensional image;
an input device for inputting data regarding the person; and
a gait and pressure capturing device constructed and arranged to obtain gait and pressure information of the body part.

15. The system of claim 14, further comprising a transmitter for transmitting the three-dimensional image, the data regarding the person and the gait and pressure information to a remote system.

16. A method of providing a custom made orthotic, the method comprising:
scanning a body part of a person and obtaining a digital scan;
inputting person information into a data input device;
measuring gait and pressure information regarding the body part;
transmitting the digital scan, person information and gait and pressure information to an analyzing device;
generating a digital three-dimensional image of the body part based on the digital scan;
analyzing the three-dimensional image based on the gait and pressure information and the person information at the analyzing device;
creating an orthotic on the model.

17. The method of claim 16, wherein scanning the body part includes generating a three-dimensional scan of the body part.

18. The method of claim 16, further comprising
obtaining balance information of the body part;
obtaining a two-dimensional image of the body part;
recording a real time video of the body part;
analyzing the gait, pressure and balance information utilizing the two-dimensional image, the three-dimensional image, and the real time video.

19. The method of claim 16, wherein transmitting the digital scan includes transmitting the digital scan via email.

20. The method of claim 16, wherein transmitting the digital scan includes uploading the digital scan into a network system.

21. The method of claim 16, further comprising modifying the three-dimensional image based.

22. The method of claim 16, further comprising receiving special instructions from the person.

23. The method of claim 16, further comprising receiving a choice of material for the custom orthotic from the person.

24. The method of claim 16, wherein measuring gait and pressure information regarding the body part includes providing a platform on which the person may stand, and providing a plurality of sensors in the platform.

25. The method of claim 24, wherein providing the plurality of sensors includes providing thin film tactile pressure and force sensors in the platform.

26. The method of claim 16, wherein measuring gait and pressure information regarding the body part includes placing an insert into the person's shoe, and providing a plurality of sensors in the insert.

27. The method of claim 26, further comprising measuring the person's balance information, wherein measuring gait, pressure and balance information includes placing providing a plurality of sensors in an insert and placing the insert into a shoe of the person, and measuring the gait, pressure and balance information of the person while the person is in motion.

28. The method of claim 16, further comprising providing a plurality of profiles and comparing the digital scan, three-dimensional image, person information inputted into the data input device, or the gait and pressure information regarding the body part to the profiles.

29. The method of claim 28, wherein the body part is a foot, the method further comprising determining whether the foot is normal, flat or high arch with respect to the profile.

30. The method of claim 16, further comprising determining and recording regions of highest pressure of the body part.

31. The method of claim 16, wherein the body part comprises a foot; the method further comprising determining and recording percentage of weight distribution of the foot while the person is standing or walking.

32. The method of claim 16, wherein the body part comprises a foot; the method further comprising determining and recording a difference in weight distribution between the forefoot and the rearfoot.

33. The method of claim 16, wherein the body part comprises a foot; the method further comprising determining and recording a difference in weight distribution between the left side and the right side of the foot.

34. The method of claim 16, wherein the body part comprises left and right feet; the method further comprising determining and recording a difference in weight distribution between the left and right feet.

35. The method of claim 16, further comprising providing devices for scanning and measuring gait and pressure of the body part and calibrating the devices.

36. The method of claim 16, further comprising conducting pre-treatment and post-treatment comparisons of the body part in motion for a person undergoing treatment.

37. The method of claim 16, wherein obtaining the three-dimensional image includes using a two-dimensional image grayscale conversion.

38. The method of claim 16, wherein obtaining the three-dimensional image includes using a computer to convert a two-dimensional image into a three-dimensional image.

39. The method of claim 16, wherein obtaining the three-dimensional image includes using a computer aided design or computer aided manufacturing technology.

40. The method of claim 16, further comprising generating a model of the body part based on the analysis.

41. The method of claim 40, wherein generating a model includes fabricating a model from a material including foam, wood and composite blanks.

42. The method of claim 16, wherein creating an orthotic includes carving the orthotic.

43. The method of claim 16, further comprising scanning the body part at a first location and creating the orthotic at a second location separate from the first location.

44. The method of claim 43, further comprising providing the orthotic at the first location for the person and evaluating proper fit of the orthotic on the person.

45. The method of claim 44, further comprising modifying the digital scan to produce a new orthotic.

46. A method of providing a custom made orthotic, the method comprising:
scanning a body part of a person;
obtaining a digital three-dimensional image of the body part;
measuring gait and pressure information regarding the body part;
analyzing the three-dimensional image based on the gait and pressure information; and
producing a custom orthotic based on the three-dimensional image and the gait and pressure information.

47. The method of claim 46, wherein producing the custom orthotic is performed at a location separate from scanning the body part.

48. The method of claim 47, further comprising transmitting the three-dimensional image and the gait and pressure information to the location for producing the custom orthotic.
